# EUROPÄISCHE PATENTANMELDUNG

(11) **EP 0 968 690 A1**
(43) Veröffentlichungstag der Anmeldung: **05.01.2000**
(21) Anmeldenummer: 98810620.9
(22) Anmeldetag: 02.07.1998
(51) Int. Cl.: A61F 2/30, A61F 2/46

(54) **Sperrsystem für den Markkanal eines Röhrenknochens**

(71) Anmelder: Sulzer Orthopädie AG, 6340 Baar (CH)
(72) Erfinder: Spotorno, Lorenzo, Prof.Dr.med., 17024 Finale Ligure (IT); Frick, Willy, Dr., 3084 Wabern (CH); Heller, Mathias, 8404 Winterthur (CH)
(74) Vertreter: Sulzer Management AG

(57) **Zusammenfassung**

Mit der Erfindung sind Sperrsysteme für den Markkanal (1) eines Röhrenknochens (2) gezeigt, welche ein Sperrelement (5) aufweisen, um Knochenzement (3) von zementierten Prothesenschäften (4) zurückzuhalten. Dadurch, dass zwischen dem Sperrelement (15) und dem Schaftende (7) ein deformierbarer und im Körper abbaubarer Pfropfen (6) aufgesetzt ist, der als Platzhalter ein Vordringen von flüssigem Knochenzement (3) verhindert, und der ein Eindringen vom Ende (7) des Prothesenschaftes erlaubt, entsteht ein Zementköcher ohne Boden, der Setzbewegungen des Schaftes im ausgehärteten Knochenzement erlaubt.

## Beschreibung

Die Erfindung handelt von einem Sperrsystem für den Markkanal eines Röhrenknochens, in welchem ein Prothesenschaft mit Knochenzement befestigbar ist, wobei das Sperrsystem ein Sperrelement aufweist, welches im Markkanal vor dem Einbringen des Knochenzements verankerbar ist, um ein Vordringen von Knochenzement zu verhindern.

Die bisherige Lehre für die Gestaltung des Zementmantels um den Schaft einer einzementierten Prothese geht von einem möglichst gleichdicken Zementmantel aus, der den Schaft vollständig umgibt, wobei allenfalls unter dem Schaftende eine etwas grössere Ansammlung von Knochenzement toleriert wird, um die Schwankungen zwischen Setztiefe von einer mechanischen Markraumsperre und Eindringtiefe vom Schaftende auszugleichen.

Eine mechanische Markraumsperre dieser Art ist in der Patentschrift US 4,293,962 mit einem zugehörigen Setzwerkzeug beschrieben. Eine ähnliche Markraumsperre zeigt die Patentschrift US 4,245,359 oder die US 4,344,190, in welcher das Material der mechanischen Markraumsperre im Körper abbaubar sein kann. Ein Nachteil einer solchen Anordnung besteht darin, dass relativ grosse Zugspannungen im Uebergang vom Zementköcher zu dem unter dem Schaftende liegenden Zementpfropfen auftreten können, wenn sich der Schaft innerhalb des ausgehärteten Zementköchers geringfügig nach unten bewegt.

Aufgabe der vorliegenden Erfindung ist es, die Zugspannungen in der Richtung der Schaftprothese im unteren Bereich des Zementköchers klein zu halten. Diese Aufgabe wird mit den Kennzeichen vom unabhängigen Anspruch 1 dadurch gelöst, dass auf dem Sperrelement ein Pfropfen aus einem deformierbaren Material aufgesetzt ist, der als Platzhalter ein Vordringen von flüssigem Knochenzement verhindert und der ein Eindringen des Endes des Prothesenschafts erlaubt.

Ein Vorteil dieser Anordnung besteht darin, dass sich das Schaftende entsprechend den Kriechbewegungen vom Knochenzement innerhalb des röhrenförmigen Köchers geringfügig absenken kann. Weiterhin ist kein Zementpfropfen vorhanden, der aufgrund einer solchen Kriechbewegung abgesprengt werden kann oder einseitig weggedrückt wird. Es ist auch kein Zementpfropfen vorhanden der bei einer Reoperation mühsam entfernt werden müsste. Ausserdem wird eine grössere Ansammlung von Knochenzement vermieden, ohne dass deswegen engere Toleranzen zwischen der mechanischen Markraumsperre und dem Schaftende eingehalten werden müssten. Dadurch, dass der Pfropfen zwischen Schaftende und mechanischer Markraumsperre deformierbar ist, kann die Schaftspitze eindringen, während der noch fliessfähige Knochenzement zurückgedrängt wird. Da der Pfropfen auch nach dem Aushärten vom Knochenzement noch verformbar ist, kann er geringfügigen Schaftabsenkungen folgen, wobei der im Knochen abgestützte Zementköcher stärker zusammengepresst wird. Die Erhöhung der ringförmig umlaufenden Zuspannung im unteren Köcher fällt dabei gering aus, da wegen des schwachen Konuswinkels in der Grössenordnung von wenigen ° eine kleine axiale Schaftabsenkung eine wesentlich geringere Durchmesservergrösserung verursacht. Vorteilhafte Weiterbildungen der Erfindung ergeben sich aus den abhängigen Ansprüchen 2 bis 15. Ein weiterer Vorteil besteht, wenn sich der deformierbare Pfropfen im Körper abbaut und auf diese Weise körpereigenes Gewebe in Form von Knochenmark und Fett bis an das Schaftende einwachsen kann. Falls sich mittelfristig auch noch das Material des Sperrelements im Körper abbaut, sind unterhalb vom Schaftende keine körperfremden Teile mehr vorhanden, um deren Abmessungen ein Reoperationsschaft tiefer gesetzt werden müsste. Ausserdem ist bei einer Reoperation nach dem Entfernen des über den Köcher vorstehenden Schaftes ein Entfernen des rohrförmigen Zementköchers wesentlich einfacher. Im weiteren wird mit dieser Anordnung verhindert, dass ein bis anhin übliche Zementpfropfen einseitig vom Köcher abgerissen wird und unter dem langsam absinkenden Schaft seitlich in den Röhrenknochen einwandert und diesen schwächt. Der deformierbare Pfropfen kann aus einem plastisch verformbaren, gallertartigen oder pasteusen Material bestehen, welches beim Eindringen einer Schaftspitze weitgehend unter Beibehaltung des Volumens ausweicht. Er kann aber auch aus einem geschäumten Material bestehen, dessen Volumen am Ort des Eindringens von einem festen Gegenstand zusammenfällt. Dies schliesst nicht aus, dass der geschäumte Pfropfen eine gewisse Elastizität hat, die es gestattet ihn auf einen kleineren Durchmesser komprimiert mit einer Setzvorrichtung in einen Markkanal einzuführen, wo er nach dem Absetzen einen grösseren Durchmesser annehmen kann. Falls man einen Schaum verwendet, der sich beim Freisetzen leicht verfestigt, lässt sich so ein Pfropfen mit einer Sprühdose und einem Verlängerungsröhrchen direkt auf dem Sperrelement absetzen und nach entsprechender Verfestigung als der erwähnte Platzhalter verwenden. Hydrolisierbare gelatinehaltige Materialien können im Körper innerhalb von Stunden bis Tagen abgebaut werden. Ebenso gibt es bioresorbierbare Materialien die der Körper mit seinen Enzymen in Tagen bis Wochen abbauen kann, sodass es mit solchen Pfropfen möglich ist unmittelbar nach der Operation, spätestens bei den ersten Belastungsversuchen unter der Schaftspitze einen Raum zu haben, der ein den Kriechbewegungen im Zementköcher entsprechendes Absinken des Schaftes erlaubt, ohne dass dabei zusätzliche Belastungsspitzen am Köcher und am Uebergang von diesem zum stützenden Knochengewebe auftreten.

Der deformierbare Pfropfen muss vor dem Einbringen des Knochenzements als Platzhalter zum Sperrelement aufgebracht werden. Ein plastisch deformierbarer Pfropfen kann auch mit einer mechanischen Markraumsperre zusammen durch eine Setzvorrichtung positioniert werden und anschliessend mit einem Stopfen gleichmässig verteilt werden. Es ist aber auch möglich, einen deformierbaren Pfropfen mit einer eigenen Setzvorrichtung abzusenken und mit einem Kolben auszustossen. Ebenso ist es möglich, den plastisch deformierbaren Pfropfen mit einer elastischen und im Körper abbaubaren Membran zu umgeben, damit er sich in Form einer länglichen Patrone in den Markraum einschieben lässt und beim Auftreffen auf die mechanische Markraumsperre den Querschnitt des Markraums gegenüber den nachfolgenden Knochenzement beansprucht. Weil der Druck vom Knochenzement zum plastisch deformierbaren Stopfen überall gleich gross ist, erfährt dieser keine wesentliche Formänderung, sondern liegt nur überall mit dem entsprechenden Druck an, während der nachfolgende Schaft zunächst den Knochenzement und anschliessend die Masse vom plastischen Pfropfen nach aussen verdrängt. Bei der Verwendung einer Membran um den Pfropfen kann es vorteilhaft sein, das Schaftende mit einer Spitze zu versehen. Diese kann aufgesteckt oder Bestandteil vom Schaft sein, um die Membran aufzustechen.

Im folgenden wird die Erfindung anhand von Ausführungsbeispielen beschrieben. Es zeigen:
- Fig. 1: Schematisch in einem Längsschnitt eine Setzvorrichtung, mit welcher ein deformierbarer Pfropfen auf einer mechanischen Markraumsperre in einem Röhrenknochen absetzbar ist;
- Fig. 2: schematisch die Anordnung von Fig. 1 beim Ausstossen und Anpressen eines deformierbaren Pfropfens;
- Fig. 3: schematisch die Anordnung von Fig. 2 nach dem Ausfahren der Setzvorrichtung und nach dem Einfüllen von fliessfähigem Knochenzement;
- Fig. 4: schematisch in einem Längsschnitt eine Setzvorrichtung für eine mechanische Markraumsperre mit einem ringförmig aufgesetzten plastisch deformierbaren Pfropfen;
- Fig. 5: schematisch die Anordnung von Fig. 4 beim Anpressen des plastisch deformierbaren Pfropfens an die verankerte Markraumsperre;
- Fig. 6: schematisch die Anordnung von Fig. 5 nach dem Ausfahren der Setzvorrichtung und nach dem Einfüllen von fliessfähigem Knochenzement;
- Fig. 7: schematisch eine Anordnung nach Fig. 3 oder 6, bei welcher ein Prothesenschaft in den deformierbaren Pfropfen hineintaucht;
- Fig. 8: schematisch die Anordnung von Fig. 7, bei der der deformierbare Pfropfen durch körpereigenes Gewebe ersetzt ist;
- Fig. 9: schematisch die Anordnung von Fig. 7, bei der der deformierbare Pfropfen und die mechanische Markraumsperre durch körpereigenes Gewebe ersetzt sind;
- Fig. 10: schematisch in einem Längsschnitt einen plastisch deformierbaren Pfropfen mit umhüllender Membran, der auf einer mechanischen Markraumsperre abgesetzt ist;
- Fig. 11: schematisch die Anordnung von Fig. 10 nach dem Einfüllen von fliessfähigem Knochenzement;
- Fig. 12: schematisch die Anordnung von Fig. 11, bei welcher ein Prothesenschaft mit einer Spitze die umhüllende Membran durchstossen hat und in den plastisch deformierbaren Pfropfen eintaucht;
- Fig. 13: schematisch die Anordnung von Fig. 12, bei der der plastisch deformierbare Pfropfen durch körpereigenes Gewebe ersetzt ist;
- Fig. 14: schematisch die Anordnung von Fig. 12, bei der der plastisch deformierbare Pfropfen und die mechanische Markraumsperre durch körpereigenes Gewebe ersetzt sind;
- Fig. 15: schematisch eine Anordnung analog zu Fig. 12, bei welcher auf einen Schaft eine Zentrierhilfe mit einer Spitze die umhüllende Membran durchstossen hat und in den plastisch deformierbaren Pfropfen eintaucht;
- Fig. 16: schematisch eine Einfüllvorrichtung in Form einer Hülse mit Ringkolben, mit welchen ein geschäumter und sich verfestigender Pfropfen auf einer mechanischen Markraumsperre anbringbar ist;
- Fig. 17: schematisch einen Schaft mit einer im unteren Drittel angeordneten Zentrierhilfe, welche das Schaftende für ein Eintauchen im deformierbaren Pfropfen frei lässt; und
- Fig. 18: schematisch eine mechanische Markraumsperre mit einer Schaftzentrierung in Form von trichterförmig angeordneten Armen, welche in einen deformierbaren Pfropfen hineinragen.

In den Figuren sind Sperrsysteme für den Markkanal 1 eines Röhrenknochens 2 gezeigt, welche ein Sperrelement 5 aufweisen, um Knochenzement 3 von zementierten Prothesenschäften 4 zurückzuhalten. Dadurch, dass zwischen dem Sperrelement 15 und dem Schaftende 7 ein deformierbarer Pfropfen 6 aufgesetzt ist, der als Platzhalter ein Vordringen von flüssigem Knochenzement 3 verhindert, und der ein Eindringen vom Ende 7 des Prothesenschaftes erlaubt, entsteht ein Zementköcher ohne Boden, der Setzbewegungen des Schaftes im ausgehärteten Knochenzement erlaubt.

In den nachfolgenden Beispielen sind gleiche Hinweiszeichen für gleiche Funktionselemente verwendet.

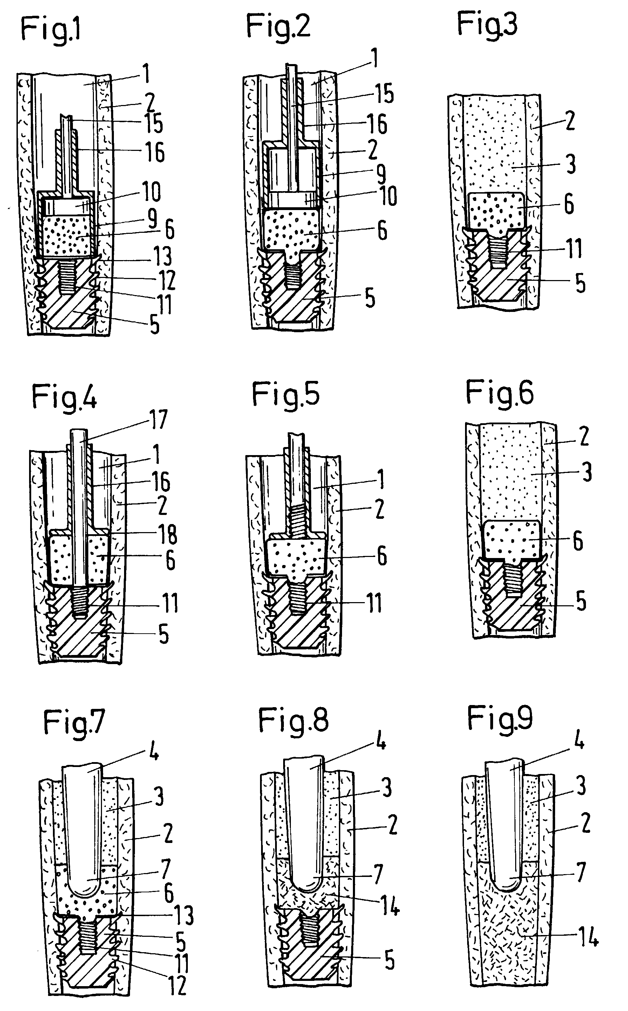

In den Figuren 1 bis 3 ist die Funktion eines Setzwerkzeuges 10, 15, 16 für einen deformierbaren Pfropfen 6 gezeigt. Dieser deformierbare Pfropfen 6 besteht aus einem plastisch deformierbaren Material, welches im menschlichen Körper abbaubar ist, wobei dieser Abbau durch Auflösung wie bei hydrolisierbarer Gelatine und/oder durch Resorption von pasteusen bioresorbierbaren Stoffen erfolgen kann. Das Setzwerkzeug weist einen Zylinder 9 auf, der als Hülse 16 fortgesetzt ist und einen Ausstosskolben 10 mit Kolbenstange 15 aufnimmt. Im zurückgezogenen Zustand des Kolbens 10 wird mit dem Zylinder 9 wie aus einem Plätzchenteig der Pfropfen ausgestochen und auf einer mechanischen Markraumsperre 5 abgesetzt (Fig. 1) und angedrückt (Fig. 2). Die Stirnfläche von Kolben 10 und Zylinderwand 9 bilden eine ebene Anpressfläche. Anschliessend kann oberhalb des Pfropfens 6 flüssiger Knochenzement 3 anliegen, wobei der Pfropfen 6 als Platzhalter das Sperrelement 5 abschirmt. Das Sperrelement 5 besitzt mehrere umlaufende Rippen 12 und vorstehende elastische Dorne 13, die eine Verankerung in einem vorbearbeiteten Markkanal erlauben. Im Zentrum ist ein Gewinde 11 angebracht, um mit einer Gewindestange das Sperrelement setzen zu können.

In den Figuren 4 bis 6 ist ein kombiniertes Setzwerkzeug 16, 17, 18 für ein gemeinsames Setzen von Sperrelement 5 und von einem ringförmigen plastisch deformierbaren Pfropfen 6. Durch eine Führungshülse 16 ragt eine Gewindestange 17, die leicht lösbar über das Gewinde 11 mit dem Sperrelement 5 verschraubt ist. Die Hülse 16 endet als Ringkolben 18. Zwischen Ringkolben 18 und dem Sperrelement 5 ist der ringförmige Pfropfen 6 gefangen und mit dem Sperrelement einbringbar. Nach dem Setzen wird die Gewindestange vom Sperrelement gelöst und bündig zum Ringkolben 18 zurückgezogen. Die so entstandene Kolbenfläche wird zum Anpressen des ringförmigen plastischen Pfropfens 6 benutzt, der noch so plastisch sein sollte, dass die mittlere Bohrung im Pfropfen 6 geschlossen wird.

Mit Fig. 6 ist dann der gleiche Zustand wie in Fig. 3 erreicht; der Pfropfen 6 wirkt als Platzhalter zwischen Knochenzement 3 und Sperrelement 5.

Eine weitere Möglichkeit einen Pfropfen 6 ohne Mittelbohrung als Platzhalter zu setzen, besteht darin ein Pfropfenmaterial zu verwenden, welches wie ein feinporiger Schwamm elastisch deformierbar ist, wobei die Porengrösse an der Oberfläche so klein ist, dass praktisch kein Knochenzement eindringt. Ein solcher "Schwamm", der sich nach einem Zusammenpressen wieder ausdehnt, wird im zusammengepressten Zustand mit einer Setzvorrichtung 10, 15, 16 gemäss Fig. 1 und 2 auf einem Sperrelement 5 abgelegt und ausgepresst, um einen Platzhalter gegen vordringenden Knochenzement zu erzeugen. Sein ursprünglicher Durchmesser ist so gross, dass er an der Innenwand des Markkanals 1 anliegt.

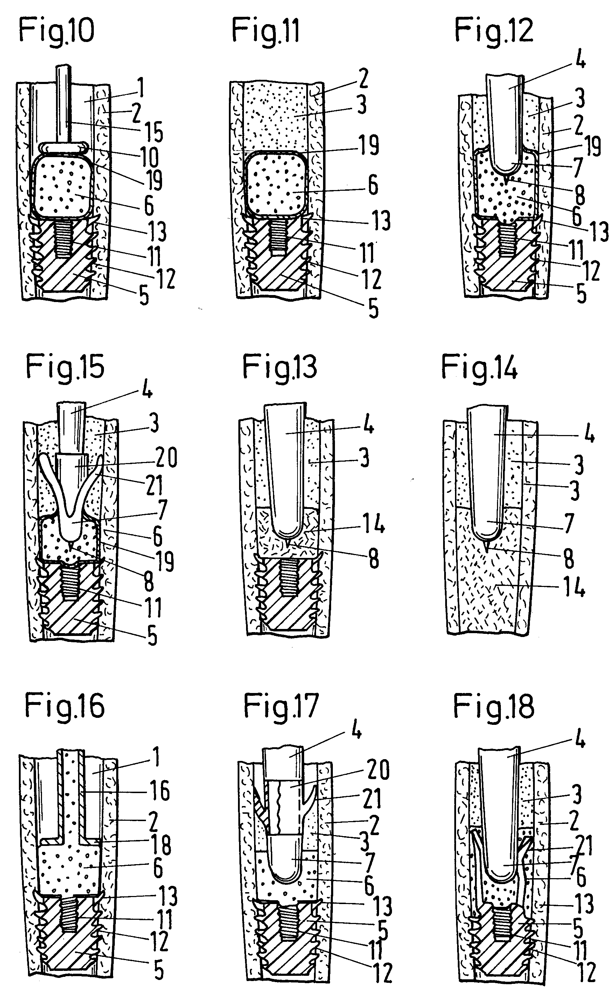

Ein Pfropfen 6 ohne Mittelbohrung lässt sich gemäss Fig. 16 auch mit einer Setzvorrichtung 16, 18 direkt auf einem Sperrelement 5 erzeugen, wenn als Pfropfenmaterial ein Schaum verwendet wird, der sich soweit verfestigt, dass er einem geringen, grossflächig anliegenden Druck von Knochenzement standhält, während er am Auftreffpunkt eines harten Schaftendes kollabiert. Das Pfropfenmaterial, welches in einer Kartusche oder Spraydose gelagert ist, wird über ein Röhrchen 16, welches sich ringförmig erweitert 18 auf einem Sperrelement 5 abgesetzt. Die Setzvorrichtung 16, 18 wird vor der Verfestigung des Schaums entfernt, damit keine Brücken zum verbleibenden Schaum im Röhrchen 16 entstehen. Nach der Verfestigung kann der so erzeugte Pfropfen als Platzhalter ein Vordringen von Knochenzement verhindern, womit ein Zustand wie in den Figuren 3 und 6 erreicht ist.

Ein flüssig an dem Pfropfen 6 anliegender Knochenzement (Fig. 3, Fig. 6) lässt ein sich absenkendes Schaftende 7 passieren, welches wie in Fig. 7 in den deformierbaren Pfropfen eindringt. Je nach Art des Pfropfens 6 reagiert dieser unterschiedlich auf das eindringende Schaftende 7. Der pasteuse, plastisch deformierbare Pfropfen 6 weicht aus und gibt einen sich einstellenden Druck wie eine eingeschlossene Flüssigkeit an die benetzten Flächen weiter. Hohlräume wie die Gewindebohrung 11 und Zwischenräume zwischen den elastischen Dornen 13 werden teilweise noch gefüllt. Um den Anteil des restlichen vom Schaftende 7 verdrängten Volumens wird die Trennfläche zwischen Pfropfen 6 und Knochenzement 3 nach oben verschoben. Bei einem einem schwammähnlichen, elastischen Pfropfenmaterial weicht das Pfropfenmaterial elastisch aus und legt sich unter erhöhter Vorspannung um das eindringende Schaftende. Ähnlich verhält sich ein elastischer, geschäumter Pfropfen 6. Ein starrer, geschäumter Pfropfen 6 kollabiert hingegen im Bereich des eindringenden Schaftendes 7 und hält den Knochenzement 3 ausserhalb des Schaftendes zurück. Das Material vom deformierbaren Pfropfen 6 ist in wenigen Stunden bis Tagen vom Körper abbaubar. Bei der Verwendung von hydrolisierbarer Gelatine als Pfropfenmaterial löst sich der Pfropfen 6 langsam in den Körperflüssigkeiten auf und verteilt sich im Körper. Bei der Verwendung von bioresorbierbaren Stoffen als Pfropfenmaterial wird der Pfropfen 6 durch körpereigene Enzyme ebenfalls in wenigen Tagen bis Wochen abgebaut und es entsteht ein Zustand gemäss Fig. 8, bei dem körpereigene Flüssigkeit und Zellen 4 den Raum zwischen Schaftende 7 und Sperrelement 5 ausfüllen, während der Köcher aus gehärtetem Knochenzement 3 wie ein abgeschnittenes Rohr endet. In diesem Zustand sind die ersten Belastungsversuche am Prothesenschaft 7 für den Zementköcher wenig gefährlich. Es können sich keine Zugspannungen über einen um das Schaftende herumlaufenden Zementpfropfen zusätzlich aufbauen und ein geringes Absenken vom Schaft 4 und Kriechbewegungen im Knochenzement sind noch möglich.

In Fig. 9 ist die Situation Monate später gezeigt, wenn sich das Sperrelement, welches bioresorbierbare Stoffe wie beispielsweise Lactide enthält, aufgelöst hat. Der Raum um das Schaftende 7 und unterhalb vom Schaftende ist mit körpereigenen Gewebe gefüllt. Ein geringfügiges Absinken vom Schaft entsprechend den grösseren Konuswinkeln im proximalen Bereich, würde nicht zu unzulässigen Zugspannungen am distalen Ende des Köchers aus Knochenzement führen. Falls später eine Reoperation nötig wird, kann der Zementköcher nach dem Herauslösen des Schaftes relativ einfach entfernt werden.

In den Figuren 15, 17 und 18 sind Anordnungen der Erfindung gezeigt, bei denen ein Schaft 4 mit seinem Ende 7 in einem deformierbaren Pfropfen 6 eintaucht und gleichzeitig eine Zentrierung innerhalb des Markkanals 1 erfährt. In Fig. 15 ist die Zentrierung dadurch gelöst, dass der Schaft 4 um eine aufsteckbare Zentrierhilfe 20 verlängert ist, deren Ende 7 in einen deformierbaren Pfropfen 6, dessen Aufbau später beschrieben wird, hineinsteht. Von der Zentrierhilfe 20 stehen seitlich elastisch federnde Arme 21 weg, mit denen der untere Teil vom Schaft 4 beim Absenken im Markkanal 1 zentriert wird. Im gezeigten Beispiel befinden sich die Arme 21 im Bereich des Knochenzements 3. Falls der deformierbare Pfropfen 6 entsprechend hoch ausgeführt wird, können auch die elastischen Arme 21 in den deformierbaren Pfropfen eintauchen, was von Vorteil sein kann, wenn auch die Zentrierhilfe aus einem bioresorbierbaren Material besteht. Der Zementköcher erfährt dann keine Unterbrüche durch die Arme 21.

Die Zentrierhilfe 20 von Fig. 17 besteht aus einem dünnwandigen Rohrstück aus körperverträglichem Kunststoff, welches über das Schaftende 7 geschoben wurde und in einem Einstich vom Schaft 4 anliegt, damit zum Knochenzement 3 eine stetige Kontur vom Schaft erhalten bleibt. Es sind lediglich mindestens drei elastische Arme 21 vorgesehen, die das Schaftende 7, welches in den deformierbaren Pfropfen eintaucht, indirekt zentrieren. Die Beispiele von Fig. 15 und 17 nehmen in Kauf, dass der noch flüssige Knochenzement 3 durch die elastische Arme 21 beim Absenken des Schaftes 4 geteilt wird und hinter den Armen wieder zusammenfliessen muss. Dieser Umstand wird im Beispiel der Zentrierung von Fig. 18 umgangen. Es sind dort am Sperrelement 5 nach oben zur Wand 2 vom Markkanal auslaufende elastische Arme 21 angebracht, die trichterförmig gegen unten zusammenlaufen und ein sich absenkendes Schaftende 7 einfangen und zentrieren können. Der deformierbare Pfropfen 6 besteht aus einem pasteusen, plastisch deformierbaren Material, das mit einer Setzvorrichtung nach Fig. 1 oder Fig. 16 nach dem Setzen des Sperrelementes 12, 21 in einer solchen Menge eingebracht und durch die elastischen Arme 21 hindurch angepresst wurde, dass die elastischen Arme 21 vollständig überdeckt sind. Nach dem Einfüllen vom Knochenzement 3 wird der Schaft 4 abgesenkt, wobei dessen Spitze 7 über die elastischen Arme 21 eingefangen und zentriert wird. Das Schaftende 7 taucht relativ weit und in den plastisch deformierbaren Pfropfen ein, damit ein langer Zentrierweg vorhanden ist und ein entsprechend grosses Volumen an flüssigem Knochenzement über das Pfropfenmaterial nach oben verschoben wird, um die mit der Zentrierung verbundene Querverschiebung der Schaftachse im Knochenzement durch den zwangsweise nach oben fliessenden Knochenzement auszugleichen.

In den Figuren 10, 11, 12, 13, 14 und 15 sind Beispiele für einen plastisch deformierbaren Pfropfen 6 gezeigt, der von einer elastischen Membran 19 umschlossen ist und der vor dem Einführen in einen Markkanal 1 eine längliche Patronenform hat. Nach dem Auftreffen des Pfropfens 6 auf einem Sperrelement 5 wird dieser mit einem Kolben 10 über eine Kolbenstange 15 angepresst (Fig. 10). Anschliessend wird der noch flüssige Knochenzement 3 eingefüllt (Fig. 11). Ein durch den Knochenzement 3 abgesenkter Schaft 4 ist an seinem Ende 7 mit einer Spitze 8 versehen, welche durch die Membran 19 in den plastisch deformierbaren Körper 6 eindringt und dessen Material verdrängt. Wenn diese Membran hochelastisch ist, benötigt man keine Spitze 8 sondern die Membran 19 legt sich dann um das eindringende Schaftende 7. Die Spitze 8 kann Bestandteil vom Schaft 4 sein. Sie kann als Spitze am distalen Ende eines Schaftes aufgesteckt sein, oder sie kann als Bestandteil einer aufsteckbaren Zentrierhilfe 20 am Schaft 4 aufgesteckt sein (Fig. 15). Die Figuren 13 und 14 zeigen analog zu den Figuren 8 und 9 ein Schaftende mit Spitze 8, das bei im Körper abbaubaren Materialien für Pfropfen 6 und Sperrelement 5 mittelfristig das am weitesten in den Röhrenknochen vorstehende Element darstellt.

## Patentansprüche

1. Sperrsystem für den Markkanal (1) eines Röhrenknochens (2), in welchem ein Prothesenschaft (4) mit Knochenzement (3) befestigbar ist, wobei das Sperrsystem ein Sperrelement (5) aufweist, welches im Markkanal (1) vor dem Einbringen des Knochenzements verankerbar ist, um ein Vordringen von Knochenzement zu verhindern, dadurch gekennzeichnet, dass auf dem Sperrelement (5) ein Pfropfen (6)aus einem deformierbaren Material aufgesetzt ist, der als Platzhalter ein Vordringen von flüssigem Knochenzement verhindert und der ein Eindringen des Endes (7) des Prothesenschafts (4) erlaubt.

2. Sperrsystem nach Anspruch 1, dadurch gekennzeichnet, dass das Material vom Pfropfen (6) im menschlichen Körper abbaubar ist.

3. Sperrsystem nach einen der Ansprüche 1 oder 2, dadurch gekennzeichnet, dass der Pfropfen (6) aus einem plastisch deformierbaren, d.h. unter dem Druck eines eintretenden Schaftendes fliessfähigem Material besteht.

4. Sperrsystem nach einem der Ansprüche 1 oder 2, dadurch gekennzeichnet, dass der Pfropfen (6) aus einem geschäumten Material besteht, welches unter dem Druck eines eintretenden Schaftendes nachgibt.

5. Sperrsystem nach einem der Ansprüche 1 bis 4, dadurch gekennzeichnet, dass der Pfropfen (6) auf ein kleineres Volumen elastisch deformierbar ist.

6. Sperrsystem nach einem der Ansprüche 1 bis 5, dadurch gekennzeichnet, dass das Material vom Pfropfen (6) im Bereich von Stunden bis mehreren Tagen im Körper abbaubar ist.

7. Sperrsystem nach einem der Ansprüche 1 bis 6, dadurch gekennzeichnet, dass das Material vom Pfropfen (6) hydrolisierbare Gelatine enthält.

8. Sperrsystem nach einem der Ansprüche 1 bis 7, dadurch gekennzeichnet, dass das Material vom Pfropfen bioresorbierbare Anteile enthält, die mit körpereigenen Enzymen abbaubar sind.

9. Sperrsystem nach einem der Ansprüche 1 bis 8, dadurch gekennzeichnet, dass das Material vom Pfropfen (6) von einer elastischen Membran umgeben ist.

10. Sperrsystem nach Anspruch 9, dadurch gekennzeichnet, dass die Membran durch eine am Ende (7) des Prothesenschaftes angebrachte scharfe Spitze (8) durchstossbar ist.

11. Setzvorrichtung für ein Sperrsystem nach einem der Ansprüche 1 bis 10, dadurch gekennzeichnet, dass sie eine zylindrische Aufnahme (9) für den Pfropfen (6) und einen in dieser gelagerten Ausstosskolben (10) aufweist, mit welchen der Pfropfen (6) in den Markkanal einbringbar ist.

12. Setzvorrichtung für ein Sperrsystem nach einem der Ansprüche 1 bis 10, dadurch gekennzeichnet, dass sie eine Kolbenfläche (10, 18) aufweist, mit der der Pfropfen an dem Sperrelement anpressbar ist.

13. Setzvorrichtung für ein Sperrsystem nach einem der Ansprüche 1 bis 8, dadurch gekennzeichnet, dass sie ein langes Röhrchen (22) aufweist, durch welches ein noch fliessfähiger Pfropfen (6) an dem Sperrelement aufsetzbar ist, wobei das Pfropfenmaterial seine Konsistenz nach dem Freisetzen so stark vergrössert, dass kein Fliessen unter Schwerkraft auftritt.

14. Sperrsystem nach einem der Ansprüche 1 bis 10, dadurch gekennzeichnet, dass das Sperrelement aus einen bioresorbierbaren Material besteht, welches im Bereich von Monaten in Körper abbaubar ist.

15. Sperrsystem nach Anspruch 14, dadurch gekennzeichnet, dass das Material des Sperrelementes Lactide aufweist.
